# FASCICULE DE BREVET EUROPEEN

(11) **EP 2 099 898 B1**
(45) Date de publication et mention de la délivrance du brevet: **28.06.2017**
(21) Numéro de dépôt: 07871814.5
(22) Date de dépôt: 07.12.2007
(51) Int. Cl.: C12N 1/04, A23K 40/30, A23K 10/18

(54) **LEVURES SÈCHES ACTIVES ENROBÉES ET ALIMENTS LES RENFERMANT**
BESCHICHTETE, AKTIVE TROCKENE HEFEN UND LEBENSMITTEL DAMIT
COATED DRIED ACTIVE YEASTS AND FOOD PRODUCTS CONTAINING THE SAME

(30) Priorité: 08.12.2006 FR 0610735
(43) Date de publication de la demande: 16.09.2009
(73) Titulaire: Danstar Ferment AG, 6300 Zug (CH)
(72) Inventeur: DEGRÉ, Richard, St-Bruno-de-Montarville, Québec J3V 3X3 (CA); BROUZES, Jérôme, F-31450 Montgiscard (FR)
(74) Mandataire: Cabinet Armengaud Aîné
(86) Numéro de dépôt international: PCT/FR2007/002019
(87) Numéro de publication internationale: WO 2008/090270

(56) Documents cités:
- EP-A1- 0 167 643
- WO-A-01/68808
- WO-A1-01/67871
- GB-A- 1 321 714
- US-A- 4 719 114

## Description

L'invention a pour objet des levures sèches actives enrobées et des aliments les renfermant.

On sait que certaines souches de levures sont utilisées comme additifs (probiotiques) dans l'alimentation des animaux.

Les aliments pour animaux sont le plus souvent commercialisés sous forme de granulés ; ces derniers sont obtenus par passage d'un mélange initialement formulé sous forme de farines ou de poudres des différents ingrédients (céréales, légumineuses, sels minéraux, vitamines etc.) dans une filière, en présence de vapeur. Cette opération provoque une élévation de la température du mélange, due à la fois à l'injection de vapeur et aux forces mécaniques liées au passage forcé dans la filière. Dans ces conditions, les levures subissent un stress qui entraîne une perte de viabilité.

Par perte de viabilité, on entend diminution du nombre de cellules capables de former des colonies (« Unités Formant Colonie », UFC) par rapport au nombre d'UFC présentes dans le mélange avant granulation. Ce nombre est représentatif de la dose d'additif probiotique « viable » dans l'aliment. Le respect de la dose recommandée est considéré par les utilisateurs comme nécessaire pour assurer l'efficacité de l'additif probiotique.

Il est donc souhaitable de limiter autant que possible, voire d'éviter totalement, cette perte de viabilité des levures au cours de la fabrication des granulés.

Pour remédier à ces inconvénients, il a été proposé de distribuer l'aliment sous forme de farine, sans granulation. Cette solution n'est toutefois pas satisfaisante, car la granulation présente par ailleurs des avantages recherchés par les fabricants et les utilisateurs (éleveurs), à savoir une meilleure digestibilité, une distribution et une consommation plus aisées, une meilleure qualité sanitaire...

D'autres solutions ont consisté à proposer d'incorporer l'additif (levures) dans les granulés refroidis. Une telle incorporation est toutefois rarement faisable en pratique, car les usines d'aliment ne sont généralement pas équipées pour ce type d'opération. De plus, il existe un risque de séparation des éléments du mélange du fait de la grande différence de taille entre granulés et particules de levures.

Dans une autre approche, il a été proposé de protéger les cellules de levures contre les contraintes thermiques et mécaniques, en enrobant les particules. Cette solution est évoquée notamment dans les documents FR 00 03409 au nom de Lallemand et WO 92 12234.

US 4719114 décrit des levures sèches actives destinées à l'alimentation qui se présentent sous forme de particules revêtues de polymère.

WO 0168808 décrit un procédé d'enrobage de microorganismes, en particulier de *Saccharomyces cerevisiae* avec de la cire de Carnauba et de *Lactobacillus casei* avec un mélange d'acide stéarique et d'acide palmitique.

WO 0167871 enseigne des levures sèches actives enrobées d'acides gras destinées à la fabrication de pâte de boulangerie ou de pâtisseries et possédant une stabilité et une capacité fermentaire accrue après que la pâte a été congelée et stockée.

La perte de viabilité demeure toutefois encore importante dans certains cas (granulation à haute température, ou à haute pression par exemple).

Les inventeurs ont cherché à apporter une solution aux problèmes évoqués ci-dessus et orienté leurs travaux sur les levures sèches. Ces levures sont le plus souvent commercialisées sous forme de « Levures Instantanées » (Instant Dry Yeast, IDY). Il existe aussi sur le marché des levures dites « Levures sèches actives » (Active Dry Yeast, ADY) obtenues par un procédé différent de séchage. Ces deux formes et procédés sont bien connus de l'Homme du Métier de la production de levures. L'aspect des particules est différent selon le procédé : cylindrique (« vermicelles ») pour IDY, sphérique (« billes ») pour ADY. De plus les IDY sont produites à partir de souches de levures réagissant plus rapidement que celles utilisées pour les ADY (Food and Agricultural Industry 1/95, pages 9.13.4-1)

Les ADY ont été élaborées pour palier les inconvénients des levures compressées disponibles jusqu'alors. Les levures compressées, qui contiennent de 26 à 32% de matière sèche, présentaient l'inconvénient de ne pas conserver leur qualité dans le temps, en particulier sous certaines conditions climatiques. Les ADY ne présentent pas cet inconvénient du fait de leur séchage et de leur taux de matière sèche supérieur à 80% et même 90%.

Toutefois, au cours du séchage qui nécessite au moins 18 heures, les ADY perdent énormément de leur activité et nécessitent une réhydratation avant utilisation par dissolution dans l'eau chaude.

De nouvelles formes de levures ont alors été proposées, à savoir les IDY (brevet US 3,843,800). La production des IDY fait appel à un autre procédé de fabrication et ne nécessite qu'un temps limité de séchage (de l'ordre de quelques dizaines de minutes). Elles présentent ainsi une activité accrue par rapport aux ADY et peuvent être mises en oeuvre telles quelles sans aucune réhydratation, notamment pour la panification et la vinification.

Il se trouve (bien que ce ne soit pas l'effet recherché à l'origine) que les ADY sont souvent plus résistantes à la granulation que les IDY. Mais cette différence est relativement modeste.

En revanche, et de manière surprenante, les inventeurs ont constaté qu'un enrobage des ADY entraînait un effet synergique sur leur résistance à la granulation et permettait de réduire significativement les pertes de viabilité des levures.

L'invention porte donc sur de nouvelles formes de levures sèches actives enrobées. Elle concerne également des aliments destinés aux animaux et comportant de telles formes de levures.

Selon un autre aspect, l'invention vise en outre un procédé de mise en oeuvre aisée pour l'obtention de telles formes de levures et d'aliments.

Les levures de l'invention sont des levures avec un revêtement protecteur d'un ou plusieurs composés inertes vis-à-vis desdites levures et sont caractérisées en ce que qu'il s'agit de levures sèches actives (ADY).

Leur revêtement est avantageusement formé de composés choisis parmi les acides gras, les sucres, les polymères naturels ou synthétiques, les protéines. Le revêtement de l'invention est un mélange d'acide stéarique et d'acide palmitique.

La proportion d'agent enrobant (ou « revêtement ») par rapport aux levures est de préférence de 10% à 90% de la masse totale du produit fini.

Lesdites levures sont plus spécialement des *Saccharomyces.*

Il s'agit plus particulièrement de *Saccharomyces cerevisiae.*

L'invention vise également des aliments pour animaux de rente ou de compagnie, caractérisés en ce qu'ils se présentent sous forme de granulés et renferment les levures telles que définies ci-dessus selon les quantités nécessaires pour obtenir l'effet probiotique recherché.

Ainsi, pour des aliments destinés à des animaux de rente, lesdites levures sont par exemple présentes à raison de 100 à 1000g/tonne, de préférence de 300 à 700 g/tonne, et notamment de l'ordre de 500 g/tonne.

Selon un autre aspect, l'invention vise un procédé d'obtention de levures telles que définies ci-dessus, caractérisé en ce qu'il comprend l'enrobage de levures sèches actives (ADY)se présentant sous forme de particules sphériques avec un ou plusieurs composés inertes vis-à-vis desdites levures, de manière à former une couche protectrice.

Les levures ainsi enrobées sont avantageusement utilisées pour préparer un aliment pour animal de rente ou de compagnie. L'invention vise donc également un procédé de préparation d'aliments pour animaux, caractérisé en ce qu'il comprend une étape de granulation d'un mélange sous forme de farine d'un aliment avec une levure telle que définie ci-dessus.

D'autres caractéristiques et avantages de l'invention seront donnés dans les exemples qui suivent de modes de réalisation.

### Exemple 1:

Une souche de levure probiotique (*Saccharomyces cerevisiae,* souche déposée à la CNCM sous le N° I-1077) a été produite selon deux procédés différents, conduisant à deux types de particules : des levures instantanées (IDY) et des levures sèches actives (ADY), ces deux procédés sont décrit par exemple dans Food and Agricultural Industry 1/95, pages 9.13.4-1 à 9.13.4-3 ou plus particulièrement pour les IDY dans US 3,843,800.

Les deux types de particules ont ensuite été enrobées avec un mélange d'acides stéarique et palmitique (50/50 % en poids) selon le procédé décrit dans FR 00 03409.

Les particules obtenues, ainsi que les particules d'ADY et d'IDY non enrobées, ont été incorporées à raison de 500 grammes par tonne dans un aliment pour bovin ayant la composition suivante :

| **Matière première** | **% en poids** |
|---|---|
| Son de blé | 35 |
| Maïs | 26 |
| Tourteau de soja | 21 |
| Mélasse de betterave | 10 |
| Luzerne déshydratée | 5 |
| Carbonate de Calcium | 1,95 |
| Prémélange de Vitamines et minéraux | 1 |
| Levure | 0,05 |

Le mélange obtenu, sous forme de farine, a été introduit dans une presse à granulation de marque Sogem, type T 90 60cv, équipée d'une filière de 4,5 mm de diamètre de passage, 45mm d'épaisseur. La température de sortie des granulés était de 85°C.
4 lots d'aliment, d'une tonne chacun, ont été fabriqués :
- un lot contenant la levure IDY non enrobée
- un lot contenant la levure ADY non enrobée
- un lot contenant la levure IDY enrobée
- un lot contenant la levure ADY enrobée

Les teneurs en levures viables (UFC) ont été analysées dans les farines et les granulés de chaque lot.

Les résultats sont présentés dans le tableau suivant :

| **Type de levure** | **CFU/g dans farine** | **CFU /g dans granulés** | **% perte de viabilité** |
|---|---|---|---|
| IDY non enrobée | 1,2x10⁷ | 2,0x10⁴ | 99,8 |
| ADY non enrobée | 1,1x10⁷ | 1,6x10⁵ | 98,6 |
| IDY enrobée | 5,6x10⁶ | 4,5x10⁵ | 92 |
| ADY enrobée | 4,9x10⁶ | 3,9x10⁶ | 20 |

L'examen de ce tableau montre que seule la levure ADY enrobée ne subit pas de forte perte de viabilité à l'issue de l'étape de granulation. La perte de viabilité ne représente en effet que 20% alors qu'elle atteint 92% avec la levure IDY enrobée. L'effet synergique de l'enrobage sur la résistance à la granulation ressort également des résultats obtenus.

### Exemple 2 :

Une souche de levure probiotique (*Saccharomyces cerevisiae ,* souche déposée à la CNCM sous le N° I-1079) a été produite selon deux procédés différents, conduisant à deux types de particules : des levures instantanées (IDY) et des levures sèches actives (ADY).

Les deux types de particules ont ensuite été enrobées avec un mélange d'acides stéarique et palmitique (50/50 % en poids) comme dans l'exemple 1 ci-dessus.

Les particules enrobées obtenues, ainsi que les particules d'ADY et d'IDY non enrobées, ont été incorporées à raison de 0.1% dans un aliment pour porc ayant la composition suivante :

| **Matière première** | **% en poids** |
|---|---|
| Orge | 38 |
| Blé | 25 |
| Tourteau de soja | 25 |
| Son de blé | 6 |
| Lard | 3 |
| Carbonate de Calcium | 1,9 |
| Prémélange de Vitamines et minéraux | 1 |
| Levure | 0,1 |

Le mélange obtenu, sous forme de farine, a été introduit dans une presse à granulation de laboratoire, de marque Kahl (type 14-175), équipée d'une filière de 4 mm de diamètre de passage. La température de sortie des granulés était de 82°C.
4 lots d'aliment, de 50kg chacun, ont été fabriqués :
- un lot contenant la levure IDY non enrobée
- un lot contenant la levure ADY non enrobée
- un lot contenant la levure IDY enrobée
- un lot contenant la levure ADY enrobée

Les teneurs en levures viables (UFC) ont été analysées dans les farines et les granulés de chaque lot.

Les résultats sont présentés dans le tableau suivant :

| **Type de levure** | **CFU/g dans farine** | **CFU /g dans granulés** | **% perte de viabilité** |
|---|---|---|---|
| IDY non enrobée | 1,84x10⁷ | 7,9x10⁵ | 95,7 |
| ADY non enrobée | 2,02x10⁷ | 1,95x10⁶ | 90,3 |
| IDY enrobée | 1,1x10⁷ | 2,15x1⁶ | 80,5 |
| ADY enrobée | 1,13x10⁷ | 9,34x10⁶ | 17,3 |

Comme dans l'exemple 1, la résistance à la granulation de la levure ADY est fortement accrue après enrobage et le pourcentage de perte de viabilité est faible comparé à celui observé avec la levure IDY enrobée.

Grâce au choix spécifique des levures ADY, l'invention fournit des moyens de grand intérêt pour obtenir des aliments ayant de remarquables propriétés probiotiques.

## Revendications

1. Aliments pour animaux de rente ou de compagnie, **caractérisés en ce qu'**ils se présentent sous forme de granulés et renferment, en tant qu'additifs probiotiques, des levures avec un revêtement protecteur de composés inertes vis-à-vis desdites levures, lesdites levures étant des levures sèches actives communément dénommées ADY et le revêtement étant un mélange d'acide stéarique et d'acide palmitique.

2. Aliments pour animaux selon la revendication 1 **caractérisés en ce que** la proportion d'agent enrobant par rapport à la levure est de 10% à 90% en poids de la masse totale du produit fini.

3. Aliments pour animaux selon l'une quelconque des revendications 1 ou 2, **caractérisés en ce que** les levures sont des *Saccharomyces.*

4. Aliments pour animaux selon l'une quelconque des revendications 1 à 3, **caractérisés en ce que** les levures Saccharomyces sont des *Saccharomyces cerevisiae.*

5. Aliments pour animaux selon l'une quelconque des revendications 1 à 4, **caractérisés en ce que** les levures se présentent sous forme de billes.

6. Aliments selon l'une quelconque des revendications 1 à 5, **caractérisés en ce que**, pour des aliments destinés à des animaux de rente, lesdites levures sont présentes à raison de 100 à 1000g/tonne, de préférence de 300 à 700 g/tonne, et notamment de l'ordre de 500 g/tonne.

7. Procédé de préparation d'un aliment pour animal de rente ou de compagnie selon l'une quelconque des revendications 1 à 6, **caractérisé en ce qu'**il comprend une étape de granulation d'un mélange, sous forme de farine, d'un aliment avec une levure avec un revêtement protecteur de composés inertes vis-à-vis desdites levures, lesdites levures étant des levures sèches actives communément dénommées ADY et le revêtement étant un mélange d'acide stéarique et d'acide palmitique.

8. Procédé de préparation d'un aliment selon la revendication 7, **caractérisé en ce que** la proportion d'agent enrobant par rapport à la levure est de 10% à 90% en poids de la masse totale du produit fini.

9. Procédé de préparation d'un aliment selon l'une quelconque des revendications 7 ou 8, **caractérisé en ce que** les levures sont des Saccharomyces.

10. Procédé de préparation d'un aliment selon l'une quelconque des revendications 7 à 9, **caractérisé en ce que** les levures Saccharomyces sont des Saccharomyces cerevisiae.

11. Procédé de préparation d'un aliment selon l'une quelconque des revendications 7 à 10, **caractérisé en ce que** les levures se présentent sous forme de billes.

12. Procédé pour réduire la perte de viabilité de levures sèches actives communément dénommées ADY pendant la granulation d'un mélange sous forme de farine d'un aliment avec une levure, **caractérisé en ce que** lesdites levures sont enrobées par un revêtement étant un mélange d'acide stéarique et d'acide palmitique.

## Patentansprüche

1. Futtermittel für Nutz- oder Haustiere, **dadurch gekennzeichnet, dass** sie in Form eines Granulats vorliegen und als probiotische Additive Hefen mit einem Schutzüberzug aus Verbindungen, die gegenüber den Hefen inert sind, umfassen, wobei die Hefen aktive Trockenhefen sind, die gewöhnlich als ADY (active dry yeasts) bezeichnet werden, und der Überzug ein Gemisch aus Stearinsäure und Palmitinsäure ist.

2. Futtermittel für Tiere gemäß Anspruch 1, **dadurch gekennzeichnet, dass** der Anteil an Überzugsmittel in Bezug auf die Hefe 10 bis 90 Gew.-% der Gesamtmasse des Endprodukts beträgt.

3. Futtermittel für Tiere gemäß einem der Ansprüche 1 oder 2, **dadurch gekennzeichnet, dass** es sich bei den Hefen um *Saccharomyces* handelt.

4. Futtermittel für Tiere gemäß einem der Ansprüche 1 bis 3, **dadurch gekennzeichnet, dass** es sich bei den *Saccharomyces-Hefen* um *Saccharomyces cerevisiae* handelt.

5. Futtermittel für Tiere gemäß einem der Ansprüche 1 bis 4, **dadurch gekennzeichnet, dass** die Hefen in Form von Kugeln vorliegen.

6. Futtermittel gemäß einem der Ansprüche 1 bis 5, **dadurch gekennzeichnet, dass** die Hefen für Futtermittel, die für Nutztiere bestimmt sind, in einem Anteil von 100 bis 1000 g/Tonne, vorzugsweise 300 bis 700 g/Tonne und insbesondere in der Größenordnung von 500 g/Tonne vorhanden sind.

7. Verfahren zur Herstellung eines Futtermittels für Nutz- oder Haustiere gemäß einem der Ansprüche 1 bis 6, **dadurch gekennzeichnet, dass** es einen Schritt des Granulierens eines Gemischs aus einem Futtermittel in Form eines Mehls mit einer Hefe mit einem Schutzüberzug aus Verbindungen, die gegenüber den Hefen inert sind, umfasst, wobei die Hefen aktive Trockenhefen sind, die gewöhnlich als ADY (active dry yeasts) bezeichnet werden, und der Überzug ein Gemisch aus Stearinsäure und Palmitinsäure ist.

8. Verfahren zur Herstellung eines Futtermittels gemäß Anspruch 7, **dadurch gekennzeichnet, dass** der Anteil an Überzugsmittel in Bezug auf die Hefe 10 bis 90 Gew.-% der Gesamtmasse des Endprodukts beträgt.

9. Verfahren zur Herstellung eines Futtermittels gemäß einem der Ansprüche 7 oder 8, **dadurch gekennzeichnet, dass** es sich bei den Hefen um *Saccharomyces* handelt.

10. Verfahren zur Herstellung eines Futtermittels gemäß einem der Ansprüche 7 bis 9, **dadurch gekennzeichnet, dass** es sich bei den *Saccharomyces-*Hefen um *Saccharomyces cerevisiae* handelt.

11. Verfahren zur Herstellung eines Futtermittels gemäß einem der Ansprüche 7 bis 10, **dadurch gekennzeichnet, dass** die Hefen in Form von Kugeln vorliegen.

12. Verfahren zur Reduktion des Verlusts an Lebensfähigkeit von aktiven Trockenhefen, die gewöhnlich als ADY (active dry yeasts) bezeichnet werden, während des Granulierens eines Gemischs aus einem Futtermittel in Form eines Mehls mit einer Hefe, **dadurch gekennzeichnet, dass** die Hefen mit einem Überzug versehen sind, der ein Gemisch aus Stearinsäure und Palmitinsäure ist.

## Claims

1. A food product for livestock or pets, **characterized in that** it is in the form of granules and contains, as probiotic additives, yeast with a protective coating made of one or more compounds which are inert relative to said yeast,
said yeast being active dry yeast commonly referred to as ADY and the coating being a mixture of stearic acid and palmitic acid.

2. The food product as claimed in claim 1, **characterized in that** the proportion of coating agent relative to the yeast is from 10% to 90% by weight of the total mass of the final product.

3. The food product according to any one of claims 1 or 2, **characterized in that** the yeast is *Saccharomyces.*

4. The food product according to any one of claims 1 to 3 **characterized in that** the *Saccharomyces* yeast is *Saccharomyces cerevisiae.*

5. The food product according to any one of claims 1 to 4 **characterized in that** the yeasts are in the form of beads.

6. The food product according to any one of claims 1 to 5 **characterized in that** for food products intended for livestock, said yeast are present in a proportion of from 100 to 1000 g/tonne, preferably from 300 to 700 g/tonne, and in particular of the order of 500 g/tonne.

7. A method for preparing a food product for livestock or pets according to any one of claims 1 to 6, **characterized in that** it comprises a step of granulating a mixture, in the form of meal, of a food product with a yeast with a protective coating made of one or more compounds which are inert relative to said yeast,
said yeast being active dry yeast commonly referred to as ADY and the coating being a mixture of stearic acid and palmitic acid.

8. A method for preparing a food product according to claim 7, **characterized in that** the proportion of coating agent relative to the yeast is from 10% to 90% by weight of the total mass of the final product.

9. A method for preparing a food product according to any one of claims 7 or 8, **characterized in that** the yeast is *Saccharomyces.*

10. A method for preparing a food product according to any one of claims 7 to 9, **characterized in that** the *Saccharomyces* yeast is *Saccharomyces cerevisiae.*

11. A method for preparing a food product according to any one of claims 7 to 10, **characterized in that** the yeasts are in the form of beads.

12. A method for reducing the viability loss of active dry yeasts commonly referred to as ADY during the granulation of a mixture, in the form of meal, of a food product with a yeast, **characterized in that** said yeasts are coated with a coating being a mixture of stearic acid and palmitic acid.
